# EUROPEAN PATENT APPLICATION

(11) **EP 1 847 274 A1**
(43) Date of publication of application: **24.10.2007**
(21) Application number: 06008283.1
(22) Date of filing: 21.04.2006
(51) Int. Cl.: A61K 45/06

(54) **Association of phenylbutyrate, ATRA and cytidine and its use in anti-tumour therapy**

(71) Applicant: CIPROSA LLC - Corporate Creations Network, Inc., Washington DC 20036 (US)
(72) Inventor: Binaghi, Gianlorenzo, 6815 Melide (CH)
(74) Representative: Trupiano, Federica

(57) **Abstract**

The present invention relates to a new association of active ingredients, in particular an association comprising a phenylbutyric acid salt, ATRA and cytidine, the pharmaceutical compositions containing said association and their use in anti-tumour therapy, optionally in combination with other anti-tumour drugs.

## Description

### Field of the invention

The present invention relates to a new association of active ingredients, in particular an association comprising a phenylbutyric acid salt, ATRA and cytidine, the pharmaceutical compositions which contain said association and their use in anti-tumour therapy, optionally in combination with other anti-tumour drugs.

### Technical background

The treatment of tumours is currently carried out by surgical removal of the tumoural mass, chemotherapy, radiotherapy or receptor therapy with monoclonal-antireceptor antibodies.

Chemotherapy is substantially carried out by administering antitumour (cytotoxic) drugs to destroy the tumour cells. Chemotherapy treatment may consist of the administration of only one drug or of several drugs. In cancer treatment, chemotherapy may be used alone or in association with surgery and/or radiotherapy. Chemotherapy is generally a very toxic treatment, badly tolerated by the patient.

Radiotherapy (also known as radiation therapy) substantially uses X rays for therapeutic purposes. The high energy used, much higher than that used for ordinary X-rays, causes the death of the tumour cells, preventing their growth phase, but it also leads to side effects causing injuries the healthy tissues close to the zone to be radiated.

Receptor therapy provides the administration of monoclonal antibodies which are combined with toxic substances and act by transporting them to the cancer cells, specifically recognising structures present on cancer cells, so it is a more selective therapy and therefore a little less toxic than chemotherapy.

However, all these therapies generally show serious problems, they are not easily tolerated by the patient and often do not lead to a real positive therapeutic result.

The object of the present invention is to provide a new therapeutic approach for the treatment of tumours, which allows the inconvenient aspects of the traditional therapies to be overcome, which is better tolerated by the patient, and which at the same time allows effective treatment of various types of tumour.

### Description of the invention

So, according to one of its aspects, the invention relates to an association of active ingredients which comprises at least one phenylbutyric acid salt, ATRA and cytidine.

According to the present invention, the expression "phenylbutyric acid salt" designates a pharmaceutically acceptable salt of said acid, for example a salt with alkaline or alkaline earth metals, for example potassium, sodium, magnesium salts, etc. or salts with organic anions such as for example amines or basic amino acids; the phenylbutyric acid sodium salt is particularly preferred.

The phenylbutyric acid sodium salt is a drug currently used as an adjuvant in the long-term treatment of the urea cycle disorders, which are essentially caused by enzymatic defects.

ATRA (an acronym for "all-trans-retinoic-acid"), also called "small effector" provitamin A, is a precursor of vitamin A which is found in nature in some vegetables.

Cytidine is a pyrimidine nucleoside which derives from the chemical demolition of the nucleic acids of which it is one of the components.

The above-mentioned compounds of the association are individually known to the art and are available on the market. Other phenylbutyric acid salts may be prepared according to methods known to the skilled in the art.

The association described above is useful in tumour therapy.

According to the present invention the term "tumours" means any neoplastic cell or mass, of any kind and origin, such as carcinomas, leukaemias, lymphomas and sarcomas.

Without wishing to be bound to particular theories, it is supposed that the effect of the association is based on the prodifferentiation capacity of the components, which act at DNA level allowing the re-expression of oncosuppressors and "tumour suppressors" that were lost due to the formation of the stable neoplastic mutation, also making them active and therefore able to induce apoptosis as the final act and inducing anoikis.

The therapeutic result of the association is mainly developed through ATRA and salified phenylbutyric acid action. ATRA belongs to the group of substances called "small effectors" (molecular weight less than 500 Daltons), and modulates the "methylation" processes at nucleosome level. The phenylbutyric acid salt carries out its molecular effect at nucleosome level with an action of modulating the "deacetylation" processes.

The combined action of ATRA and of the salified phenylbutyric acid at DNA level generates a temporary chemical instability of the H2A H2B H3 H4 non-H1 histones. This leads to a handling of the DNA around the histone octamer (200 pairs of nucleotide bases of which about 50 can be more easily handled) with consequent re-expression of genic structures which may be verified partly by the appearance of chromosome configurations which indicate the occurrence of a differentiating effect (restriction of chromatin) with likely modulation of the promoters, transcription factors and MIR.

Among the re-expressed structures there is the gene of the P53 protein, a key element in the induction of apoptosis, a mechanism that is lost in neoplastic cells.

It is supposed that cytidine strengthens a demethylation process that is necessary for the functional restoration of P53.

The combined action of ATRA and of the phenylbutyric acid salt would also result in the destructuration of the cytoskeleton, which is also involved in the induction of apoptosis and of anoikis.

So, according to another of its aspects, the invention relates to the use of the association described above for the preparation of a medicament directed to treat tumours, of any kind and origin, in a mammal, advantageously, but not exclusively, in human beings.

In some cases, the treatment according to the invention may be of the prophylactic type, when used for example in the prevention of relapses and/or in the prevention of tumours in specific subjects at risk, for example in subjects undergoing therapies which may cause the onset of tumours or who are exposed to risk factors in the environment or at work, etc.

Even where not specifically indicated, the expression "treatment", according to the present invention, therefore indicates a curative or prophylactic treatment.

Likewise, when speaking of "therapy" using the association of the invention, the term indicates a curative or prophylactic therapy.

The association of the invention may be administered along with other conventional anti-tumour treatments.

It has in fact been observed that the association of the invention limits the occurrence of M.D.R. (Multi-Drug Resistance) in conventional anti-tumour treatments.

Moreover, it has been observed that the association of the invention used in combination with radiotherapy limits the metastasising effect of thermal stress.

Lastly, it has been observed that the association of the invention used in combination with receptor therapies, besides limiting M.D.R., strengthens the effects of those therapies, favouring apoptosis after proliferative receptor block.

It is clear that all these synergies significantly improve the therapeutic result obtained.

According to another of its aspects, the invention relates to a medicament that comprises the association of the invention.

According to another of its aspects, the invention relates to a method for treating tumour which comprises administering an effective amount of the association of the invention to a subject in need thereof.

To carry out a therapy with the association of the invention, it is possible to administer the three components simultaneously or in sequence, by means of only one or of several pharmaceutical compositions, combined with pharmaceutically acceptable excipients and carriers.

So, for example, pharmaceutical compositions including all three components of the association, combined with pharmaceutically acceptable excipients and carriers, may be formulated and administered to the patient.

Alternatively, the different components of the association may be formulated in several compositions, comprising only one or two of the components, to be administered simultaneously or in sequence.

By "simultaneously" it is meant that the three components of the association are administered substantially at the same time.

By "in sequence" it is meant that the three components of the association are administered within a certain space of time, for example within a period of one hour or in 24 hours.

These compositions may be formulated for oral, intravenous, intramuscular or transdermal administration, etc.

The pharmaceutical compositions comprising the association of a phenylbutyric acid salt, advantageously the phenylbutyric acid sodium salt, ATRA and cytidine, represent a further subject-matter of the present invention.

According to a preferred aspect, the compositions of the invention comprise from 10 to 1000 mg of a phenylbutyric acid salt (based on the amount of the free acid), preferably from 50 to 500 mg, for example 220-250 mg; from 2 to 50 mg of ATRA, preferably from 5 to 10 mg, for example from 6 to 8 mg; and from 10 to 500 mg of cytidine, preferably from 50 to 300 mg, for example from 75 to 150 mg.

A particularly preferred composition is a unit dosage form, for example a solid form, which comprises about 250 mg of phenylbutyric acid sodium salt (about 220 mg of free acid), about 7 mg of ATRA and about 100 mg of cytidine.

The treatment according to the invention may comprise the administration of the association of the invention one or more times a day, for example the administration of one or more unit dosage forms, from one to four times a day.

The association of the invention has been tested in pharmacological and clinical assays which have demonstrated its efficacy in tumour therapy and have given surprising results.

The following example illustrates the invention without limiting it in any way.

### Example 1

Oral composition.

A tablet is prepared comprising:
250 mg of phenylbutyric acid sodium salt
7 mg of ATRA
100 mg of cytidine
in combination with pharmaceutically acceptable excipients.

## Claims

1. Association of active ingredients comprising at least one phenylbutyric acid salt, ATRA and cytidine.

2. Association according to claim 1, **characterised in that** said phenylbutyric acid salt, is selected from a salt with alkaline or alkaline earth metals and a salt with organic anions.

3. Association according to claim 2, **characterised in that** said salt is phenylbutyric acid sodium salt.

4. Association according to any one of claims 1 to 3, for its use in therapy.

5. Association according to claim 4, for its use in tumour therapy:

6. Association according to claims 4 or 5, which is formulated in only one or in several pharmaceutical compositions.

7. Association according to claim 6 which is formulated in several pharmaceutical compositions to be administered simultaneously or in sequence.

8. Medicament comprising the association of any one of claims 1 to 7.

9. Pharmaceutical composition comprising the association of any one of claims 1 to 7, combined with one or more pharmaceutically acceptable excipients.

10. Composition according to claim 9, **characterised by** comprising from 10 to 1000 mg of a phenylbutyric acid salt (based on the amount of the free acid), from 2 to 50 mg of ATRA and from 10 to 500 mg of cytidine.

11. Composition according to claim 10, **characterised by** comprising from 220 to 250 mg of a phenylbutyric acid salt (based on the amount of the free acid), from 5 to 10 mg of ATRA and from 50 to 300 mg of cytidine.

12. Composition according to claim 11, **characterised by** comprising 250 mg of phenylbutyric acid sodium salt (about 220 mg of free acid), 7 mg of ATRA and 100 mg of cytidine.

13. Use of the association according to any one of claims 1 to 7, for the preparation of a medicament directed to tumour therapy.

14. Use of a medicament according to claim 8 or of the compositions of the claims from 9 to 12 for the preparation of a medicament directed to tumour therapy.

15. Use according to any one of claims 13 or 14, in combination with other anti-tumour therapies.
